# EUROPEAN PATENT APPLICATION

(11) **EP 4 342 951 A1**
(43) Date of publication of application: **27.03.2024**
(21) Application number: 22197310.0
(22) Date of filing: 23.09.2022
(51) Int. Cl.: C09B 23/04, C09B 23/10, C09B 57/00, G01N 33/50

(54) **IMIDAZO[1,2-A]PYRIDINIUM DERIVATIVES**

(71) Applicant: Universität Wien, 1010 Wien (AT)
(72) Inventor: MAULIDE, Nuno, 1090 Vienna (AT); RIOMET, Margaux, 1090 Vienna (AT); SARIDAKIS, Iakovos, 1090 Vienna (AT); RUKAVINA, Stefanie, 1090 Wien (AT)
(74) Representative: Gassner, Birgitta

(57) **Abstract**

The present invention relates to novel compounds of general formula I 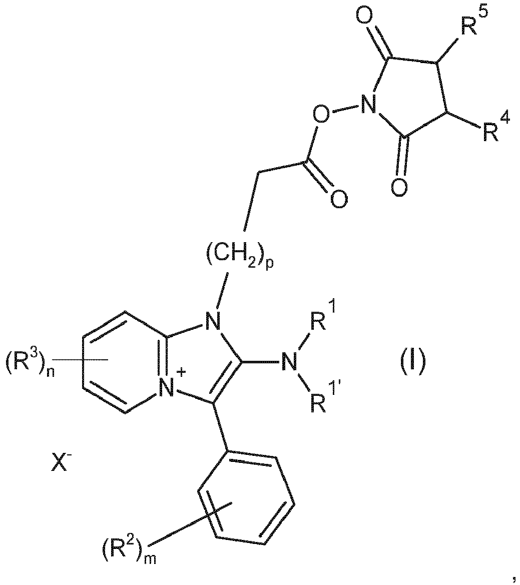 wherein X⁻, R¹ - R⁵, n, m, and p have the meanings given in the description and claims, process for preparing these compounds and use thereof.

## Description

### Field of the Invention

The present invention relates to novel compounds of general formula I 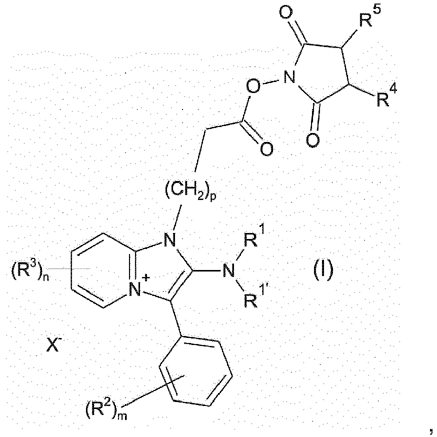 wherein X⁻, R¹-R⁵ and n, m, and p have the meanings given in the description and claims, process for preparing these compounds and use thereof

### Background Art

Fluorescent probes are powerful tools that are widely used in various research fields such as biology, biochemistry, chemical biology and medical diagnostics. Among their multiple usages, they are used to identify protein location and their conformational changes and to monitor *in vivo* processes.

Several families of fluorescent dyes have been developed and commercialized in the past few years covering the whole color spectra. They are designed to be easily cross-linked to the molecules or proteins of interest commonly via amide coupling or click chemistry.

Imidazo-pyridines have also been reported to be fluorescence active. The imidazo-pyridine core is found in many drugs and biologically active molecules (Kazmierczak, A. et al., J. Med. Chem. 2017, 60, 8781; Silva, D. G. et al., ACS Med. Chem. Lett. 2017, 8, 766; Sayeed, I. B. et al., Med. Chem. Commun. 2017, 8, 1000; Enguehard-Gueiffier, C. and Gueiffier, A. Mini-Rev. Med. Chem. 2007, 7, 888). As the neutral heterocycles, the corresponding salts do not only display biological activity but also proved to be fluorescence active. In the last decade, several research groups described the synthesis of imidazo-pyridines with interesting photochemical properties. They can be divided into two subcategories, the one embedding an imidazo[1,5-a] pyridine core and the one embedding an imidazo[1,2-a]pyridine core.

A novel route to *b*-fused thiazole compounds starting from a phenacylpyridinium salt and KSCN is described by Babaev E. V. et al. (Russian chemical Bulletin International Edition Seriya Khimicheskaya, vol 171 (1), 2005, pp 231-237). The various activities of aminothiazolopyridinium salts were tested and described by Babaev E. V. et al. (Tetrahedron Letters, vol 40(42), 1999, pp 7553-7556). Zhang Tongxin et al. (Dyes and Pigments, vol 171, 2019) have disclosed fluorescence-enhanced chemosensors for imaging living cells in extreme acidity. A new family of azonia aromatic heterocycles comprising imidazopyridinium cations and their use as DNA intercalators is described by Bosch P. et al. (Dyes and Pigments, vol 138, 2016, pp 135-146).

However, most of the so far developed fluorescent dyes show some limitations. They are synthetically challenging to make, poorly modular and are relatively expensive for the final customer. Thus, there is still a need for new and modular fluorescent dyes and approaches for synthesizing them. It is an aim of the present invention to provide a new pyridinium core with photophysical properties tunable over the whole color spectrum by post functionalization of the core.

### Summary of invention

It has surprisingly been found, that compounds of general formula I, wherein X-, R¹-R⁵, n, m, and p have the meanings below, exhibit photophysical properties and show fluorescent activity. Their photophysical properties can be easily tuned to cover the whole color spectrum by post functionalization of the pyridinium core and they can be easily functionalized to be later cross-linked to the molecules or proteins of interest. Described is also a modular and combinatorial synthesis of fluorescent polysubstituted imidazo[1,2-a]pyridinium salts and their analogs.

Various objects, features, aspects, and advantages of the present invention will become more apparent from the following detailed description of embodiments of the invention.

### Description of embodiments

The present invention relates to compounds of general formula (I), 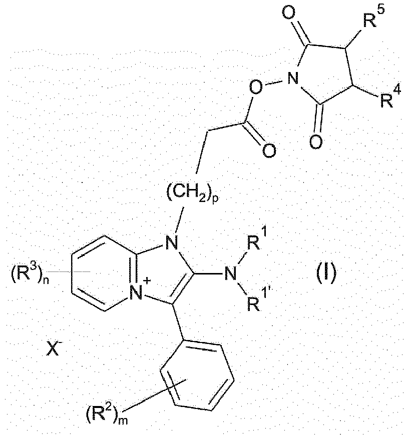 wherein
X- denotes an anion,
R¹ denotes C₁₋₁₀alkyl, C₂₋₁₀alkenyl, or phenyl, optionally substituted by one or more, identical or different R^{a} and/or R^{b}, and
R^{1'} denotes H, C₁₋₆alkyl, or C₂₋₆alkenyl, optionally substituted by one or more, identical or different R^{a} and/or R^{b}, or
R¹ together with R^{1'} form a 3 to 12 membered heterocyclic ring, and
R² denotes H, halogen, -SCR^{a}, -NO₂, -N3, -NR^{a}R^{a}, -P(O)(OR^{a})(OR^{a}), -S(O)₂OR^{a},
-OR^{a}, -C(O₂)R^{a}, or C₁₋₆alkyl, C₆₋₁₀aryl, optionally substituted by one or more, identical or different R^{a} and/or R^{b}, , and
R³ denotes H, halogen, -SCR^{a}, -NO₂, -N3, -NR^{a}R^{a}, -P(O)(OR^{a})(OR^{a}), -S(O)₂OR^{a},
-OR^{a}, -C(O₂)R^{a}, or C₁₋₁₀alkyl, C₂₋₁₀alkenyl, phenyl, C₆₋₁₄aryl, 5-14 membered heteroaryl, or 5-14 membered heterocyclyl, optionally substituted by one or more, identical or different R^{a} and/or R^{b}, or
two R³ may be bond to each other to form an aromatic ring, and R⁴ and R⁵ independently from each other denote H, or halogen, or
R⁴ and R⁵ may be bond to each other to form an optionally substituted 5- to 6-membered cyclic group, and
m and n independently from one another denote 0, 1, 2, 3, or 4, and p denotes 2, 3, 4, 5
each R^{a} independently of one another denotes H, or a group, optionally substituted by one or more, identical or different R^{b} and/or R^{c}, selected from among C₁₋₁₀alkyl, phenyl, and 3-14 membered heterocyclyl; and
each R^{b} is selected in each case independently of one another from among halogen, - CF₃, -NO₂, =N₂, -N₃, -NR^{c}R^{c}, =O, -OR^{c}, -C(O)R^{c}, -C(O)OR^{c}, -C(O)NR^{c}R^{c}, -P(O)(OR^{c})(OR^{c}), -SCR^{c}, -S(O)₂OR^{c}, and -S(O)₂R^{c}, and
each R^{c} independently of one another denotes hydrogen or a group, optionally substituted by one or more, identical or different R^{d} and/or R^{e}, selected from among C₁₋₁₀alkyl, C₂₋₁₀alkenyl, phenyl, and 3-18 membered heterocyclyl;
each R^{d} denotes a suitable substituent and is selected in each case independently of one another from among halogen, CF₃, -NO₂, =N₂, -N₃, -NR^{c}R^{c}, =O, -OR^{c}, -C(O)R^{c}, - C(O)OR^{c}, -C(O)NR^{c}R^{c}, -P(O)(OR^{c})(OR^{c}), -SCR^{c}, -S(O)₂OR^{c}, and -S(O)₂R^{c}; and
each R^{e} independently of one another denotes hydrogen or a group selected from among C₁₋₁₀alkyl, C₂₋₁₀alkenyl, phenyl, and 3-18 membered heterocyclyl.

A further embodiment of the invention relates to compounds as described herein, wherein R¹ together with R^{1'} form a 5 membered heterocyclic ring.

A further embodiment of the invention relates to compounds as described herein, wherein R³ is a 5-14 membered heterocyclyl.

A further embodiment of the invention relates to compounds as described herein, wherein R³ is tetrahydro-pyrido-quinoline moiety.

A further embodiment of the invention relates to compounds as described herein, wherein m denotes 0.

A further embodiment of the invention relates to compounds as described herein, wherein p denotes 3.

A further embodiment of the invention relates to compounds as described herein, wherein X- denotes a chloride, bromide, iodide, fluoride, acetate, formiate, perchlorate, fluorinated alkyl sulfonate or fluorinated aryl sulfonate, trifluorosulfonate, trifluoromethanesulfonate (triflate), nonafluorobutanesulfonate (nonaflate), halogenated carboxylate, trifluoroacetate, trichloroacetate, dichloroacetate, monochloroacetate, tetrafluoroborate, hexafluorophosphate, or hexafluoroantimonate.

A further embodiment of the invention relates to compounds as described herein, wherein X- denotes trifluoromethanesulfonate (triflate).

One embodiment of the invention relates to compounds selected from Table 1.

| | | |
|---|---|---|
| 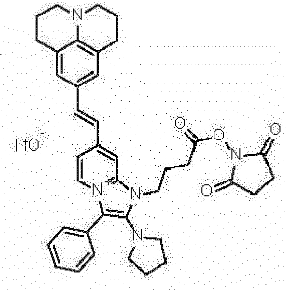 | 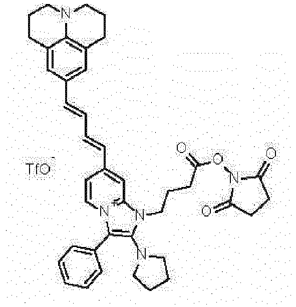 | 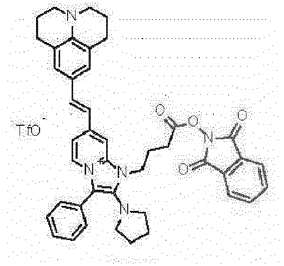 |
| I-a | I-b | I-c |

One embodiment of the invention relates to compounds as described herein, wherein the compounds exhibit photophysical properties, preferably fluorescence activity.

One embodiment of the invention relates to a process for manufacturing a compound of formula (I), wherein compounds 1 and 2 are reacted in the presence of a coupling agent, a base and a solvent to obtain a compound of formula (I), 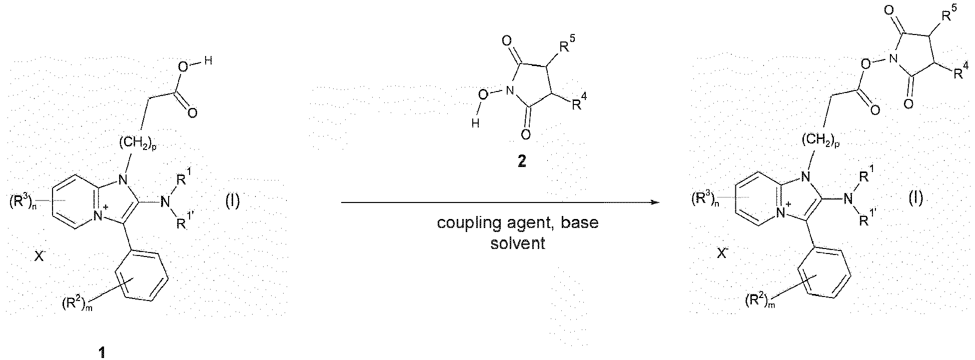 wherein
R¹, R^{1'}, R², R³, R⁴, R⁵, X⁻, m, n and p are as defined above.

The invention provides use of the compounds for the use as a fluorescent probe.

Further, the invention provides use of the compounds as a fluorescent probe, wherein the fluorescent probe is used in the field of biology, biochemistry, chemical biology, or medical diagnostics.

### Definitions

As used herein, the following definitions apply, unless stated otherwise:

Unless specified otherwise, the term "alkyl", when used alone or in combination with other groups or atoms, refers to a saturated straight or branched chain consisting solely of 1 to 6 hydrogen-substituted carbon atoms, and includes methyl, ethyl, propyl, isopropyl, n-butyl, 1-methylpropyl, isobutyl, t-butyl, 2,2-dimethylbutyl, 2,2-dimethylpropyl, n-pentyl, 2-methylpentyl, 3-methylpentyl, 4-methylpentyl, n-hexyl and the like.

Unless specified otherwise, the term "alkenyl" refers to a partially unsaturated straight or branched chain consisting solely of 2 to 6 hydrogen-substituted carbon atoms that contains at least one double bond, and includes vinyl, allyl, 2-methylprop-1-enyl, but-1-enyl, but-2-enyl, but-3-enyl, buta-1,3-dienyl, penta-1,3-dienyl, penta-2,4-dienyl, 2-methylbut-1-enyl, 2-methylpent-1-enyl, 4-methylpent-1-enyl, 4-methylpent-2-enyl, 2-methylpent-2-enyl, 4-methylpenta-1,3-dienyl, hexen-1-yl and the like.

Unless specified otherwise, the term "cycloalkyl", when used alone or in combination with other groups or atoms, refers to monocyclic hydrocarbon rings, bicyclic hydrocarbon rings or spirohydrocarbon rings, which each may be either saturated or unsaturated (cycloalkenyl). The term unsaturated means that in the ring system in question there is at least one double bond, but no aromatic system is formed. In bicyclic hydrocarbon rings, two rings are linked such that they have at least two carbon atoms in common. In spirohydrocarbon rings, one carbon atom (spiroatom) is shared by two rings. If a cycloalkyl is substituted, the substitution may be mono- or polysubstitution in each case, at all the hydrogen-carrying carbon atoms, independently of one another. Cycloalkyl itself may be linked to the molecule as substituent via any suitable position of the ring system.

Typical examples of individual sub-groups are listed below.

Monocyclic saturated hydrocarbon rings: cyclopropyl; cyclobutyl; cyclopentyl; cyclohexyl; cycloheptyl, etc.

Monocyclic unsaturated hydrocarbon rings: cycloprop-1-enyl; cycloprop-2-enyl; cyclobut-1-enyl; cyclobut-2-enyl; cyclopent-1-enyl; cyclopent-2-enyl; cyclopent-3-enyl; cyclohex-1-enyl; cyclohex-2-enyl; cyclohex-3-enyl; cyclohept-1-enyl; cyclohept-2-enyl; cyclohept-3-enyl; cyclohept-4-enyl; cyclobuta-1,3- dienyl; cyclopenta-1,4-dienyl; cyclopenta-1,3-dienyl; cyclopenta-2,4-dienyl; cyclohexa-1,3- dienyl; cyclohexa-1,5-dienyl; cyclohexa-2,4-dienyl; cyclohexa-1 ,4-dienyl; cyclohexa-2,5- dienyl, etc. Saturated and unsaturated bicyclic hydrocarbon rings: bicyclo[2.2.0]hexyl; bicyclo[3.2.0]heptyl; bicyclo[3.2.1]octyl; bicyclo[2.2.2]octyl; bicyclo[4.3.0]nonyl (octahydroindenyl); bicyclo[4.4.0]decyl (decahydronaphthalene); bicyclo[2,2,1]heptyl (norbornyl); (bicyclo[2.2.1]hepta-2,5-dienyl (norborna-2,5-dienyl); bicyclo[2,2,1]hept-2-enyl (norbornenyl); bicyclo[4.1.0]heptyl (norcaranyl); bicyclo- [3.1.1]heptyl (pinanyl), etc.
Saturated and unsaturated spirohydrocarbon rings: spiro[2.5]octyl, spiro[3.3]heptyl, spiro[4.5]dec-2-ene, etc.

Unless specified otherwise, the term "aryl" refers to an aromatic mono- or bicyclic group containing from 6 to 14 carbon atoms, preferably 6 to 10 carbon atoms, that may be optionally fused with a fully or partially saturated or unsaturated carbocyclic ring and may optionally be substituted with one or more, identical or different substituents, suitably one to three substituents. Examples of aryl groups include phenyl, naphthyl, indanyl, and the like.

Unless specified otherwise, the term "heterocyclic ring" refers to aromatic and non-aromatic cyclic rings such as for example to heteroaryl and heterocyclyl as defined herein.

Unless specified otherwise, the term "heteroaryl" refers to an aromatic mono- or bicyclic group containing from 5 to 14 carbon atoms, preferably 5 to 12 carbon atoms, of which one to five is replaced with a heteroatom selected from N, S and O, that may optionally be reduced to a non-aromatic heterocycle and may optionally be substituted with one or more, identical or different substituents. Examples of heteroaryl groups include pyrrolyl, dihydropyrrolyl, pyrrolidinyl, oxopyrrolidinyl, indolyl, isoindolyl, indolizinyl, imidazolyl, pyrazolyl, benzimidazolyl, imidazo(1,2-a)pyridinyl, indazolyl, purinyl, pyrrolo(2,3-c)pyridinyl, pyrrolo(3,2-c)pyridinyl, pyrrolo(2,3-b)pyridinyl, pyrazolo(1,5-a)pyridinyl, 1,2,3-triazolyl, 1,2,4-triazolyl, tetrazolyl, oxazolyl, 1,2 oxazolyl, isoxazolyl, 1,3,4-oxadiazolyl, 1,2,5-oxadiazolyl, 1,2,4-oxadiazolyl, 1,2,3-oxadiazolyl, thiazolyl, isothiazolyl, 1,3,4-thiadiazolyl, 1,2,5-thiadiazolyl, 1,2,4-thiadiazolyl, 1,2,3-thiadiazolyl, furanyl, dihydrofuranyl, tetrahydrofuranyl, benzofuranyl, isobenzofuranyl, thiophenyl, dihydrothiophenyl, tetrahydrothiophenyl, benzothiophenyl, benzoisothiophenyl, pyridyl, piperidinyl, quinolinyl, isoquinolinyl, tetrahydroisoqinolinyl, quinolizinyl, pyrazinyl, pyridazinyl, pyrimidinyl, pyranyl, tetrahydropyranyl, 1,2,3-triazinyl, 1,2,4-triazinyl, 1,3,5-triazinyl, chromenyl, morpholinyl, diazepinyl, benzodiazepinyl, and the like.

By the term "heterocyclyl" are meant groups which are derived from cycloalkyl as hereinbefore defined if in the hydrocarbon rings one or more of the groups -CH₂-are replaced independently of one another by the groups -O-, -S- or -NH- or one or more of the groups =CH- are replaced by the group =N-, while not more than five heteroatoms may be present in total, there must be at least one carbon atom between two oxygen atoms and between two sulphur atoms or between one oxygen and one sulphur atom and the group as a whole must be chemically stable. Heteroatoms may simultaneously be present in all the possible oxidation stages (sulphur -> sulphoxide - SO-, sulphone -SO₂-; nitrogen -> N-oxide). It is immediately apparent from the indirect definition/derivation from cycloalkyl that heterocyclyl is made up of the sub-groups monocyclic hetero-rings, bicyclic hetero-rings and spirohetero-rings, while each subgroup can also be further subdivided into saturated and partially unsaturated heterocyclyl groups. The term partially unsaturated means that in the ring system in question there is at least one double bond, but no aromatic system is formed. In bicyclic hetero-rings two rings are linked such that they have at least two atoms in common. In spirohetero-rings one carbon atom (spiroatom) is shared by two rings. If a heterocyclyl is substituted, the substitution may be mono- or polysubstitution in each case, at all the hydrogen-carrying carbon and/or nitrogen atoms, independently of one another. Heterocyclyl itself as substituent may be linked to the molecule via any suitable position of the ring system.

The term "heterocyclyl group" as used herein also refers to a heterocyclyl group as defined above, which optionally may be fused to an aromatic aryl or heteroaryl group.

Typical examples of individual sub-groups are listed below:
Monocyclic heterorings (saturated and unsaturated): oxolane, pyrrolidinyl, pyrrolinyl, imidazolidinyl, thiazolidinyl, imidazolinyl, pyrazolidinyl, pyrazolinyl, piperidinyl, piperazinyl, oxiranyl, aziridinyl, azetidinyl, 1,4-dioxanyl, azepanyl, diazepanyl, morpholinyl, thiomorpholinyl, homomorpholinyl, homopiperidinyl, homopiperazinyl, homothiomorpholinyl, thiomorpholinyl-S-oxide, thiomorpholinyl-S,S-dioxide, 1,3-dioxolanyl, oxane, tetrahydrothiopyranyl, 1,4-oxazepanyl, tetrahydrothienyl, homothiomorpholinyl-S,S-dioxide, oxazolidinonyl, dihydropyrazolyl, dihydropyrrolyl, dihydropyrazinyl, dihydropyridyl, dihydro-pyrimidinyl, dihydrofuryl, dihydropyranyl, tetrahydrothienyl-S-oxide, tetrahydrothienyl-S,S-dioxide, homothiomorpholinyl-S-oxide, 2,3-dihydroazet, 2*H*-pyrrolyl, 4*H*-pyranyl, 1,4- dihydropyridinyl, etc;
Bicyclic heterorings or polycyclic heterorings (saturated and unsaturated): 8-azabicyclo[3.2.1]octyl, 8-azabicyclo[5.1.0]octyl, 2-oxa-5-azabicyclo[2.2.1]heptyl, 8-oxa-3-aza-bicyclo[3.2.1]octyl, 3,8-diaza-bicyclo[3.2.1]octyl, 2,5-diaza-bicyclo-[2.2.1]heptyl, 1-aza-bicyclo[2.2.2]octyl, 3,8-diaza-bicyclo[3.2.1]octyl, 3,9-diaza-bicyclo[4.2.1]nonyl, 2,6-diaza-bicyclo[3.2.2]nonyl, hexahydro-furo[3,2-b]furyl, 1H-thieno[3,4-*d*]imidazol, hexahydro-1H-thieno[3,4-*d*]imidazol, saturated or unsaturated cyclopentaphenanthrene, etc;
Spiro-heterorings (saturated and unsaturated): 1,4-dioxa-spiro[4.5]decyl; 1-oxa-3,8-diaza-spiro[4.5]decyl; 2,6-diaza-spiro[3.3]heptyl; 2,7-diaza-spiro[4.4]nonyl; 2,6-diaza-spiro[3.4]octyl; 3,9-diaza-spiro[5.5]undecyl; 2,8-diaza- spiro[4.5]decyl, etc.

By the term "suitable substituent" is meant a substituent that on the one hand is fitting on account of its valency and on the other hand leads to a system with chemical stability.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques known in the art as well as those methods set forth herein.

The term "tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base.

It is also to be understood that compounds (e.g., dihydro bases described herein) that have the same molecular formula but differ in the nature or sequence of bonding of their atoms or the arrangement of their atoms in space are termed "isomers". Isomers that differ in the arrangement of their atoms in space are termed "stereoisomers".

Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomers respectively).

A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The term "hydrate" refers to a compound which is associated with water. Typically, the number of the water molecules contained in a hydrate of a compound is in a definite ratio to the number of the compound molecules in the hydrate.

Any formula or structure given herein, including Formula I compounds, is also intended to represent unlabeled forms as well as isotopically-labeled forms of the compounds. Isotopically-labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, and chlorine, such as, but not limited to 2H (deuterium, D), 3H (tritium), 11C, 13C, 14C, 15N, 18F, 31P, 32P, 35S, 36CI, and 125J.

The term "leaving group" has the meaning conventionally associated with synthetic organic chemistry, i.e., an atom or a group capable of being displaced by a nucleophile and includes halo (such as chloro, bromo, and iodo), alkanesulfonyloxy, arenesulfonyloxy, alkylcarbonyloxy (e.g., acetoxy), arylcarbonyloxy, mesyloxy, tosyloxy, trifluoromethanesulfonyloxy, aryloxy (e.g., 2,4-dinitrophenoxy), methoxy, N,O-dimethylhydroxylamino, and the like. Specific examples of leaving groups include, but are not limited to, Cl, Br, I and sulfonate esters such as OMs (mesylate), OTs (tosylate), ONs (nosylate), OTf (triflate) and any other sulfonate esters.

The term "electrophile", "electrophile molecule", or a reactant comprising an electrophilic moiety is a compound which is at least partially formed of C- and H-Atoms and comprises an electron-poor site which may react with another molecule comprising a site with an increased electron density. The "electrophile" typically carries a positive charge or a positive partial charge. In principle, various types of electrophiles or reactants comprising an electrophilic moiety can be used. In particular, as an electrophile, a Lewis-acidic organic or inorganic reactant with an electron poor site can be used. Exemplary electrophiles are molecules selected from the group consisting of triflic anhydride, PCl₃, and (COCl)₂.

The term "thiation agent" describes for example, any agent known in the art for the conversion of an oxo group to a thioxo group such as, for example, 2,4-bis-(4-methoxyphenyl)-1,3-dithia-2,4-diphosphetane-2,4-disulphide (Lawesson's Reagent) or P₂S₅ (phosphorus pentasulphide). The thiation reaction is generally carried out with the required stoichiometric amount of thiation reagent in order to reduce the risk of damage to other functional groups. In general, the reaction is carried out in a suitable solvent or diluent such as toluene, xylene or tetrahydrofuran and at a temperature, for example, at or near the reflux temperature of the solvent or diluent, that is in the range 65 to 150°C.

The term "anion" means an ionic compound preferably inorganic comprising one or several negative charges. Anion denotes, for example, an organic or inorganic anion, such as halogen, preferably chloride and fluoride, sulfate, hydrogen sulfate, phosphate, boron tetrafluoride, carbonate, bicarbonate, oxalate or alkyl sulfate, especially methyl sulfate or ethyl sulfate; anion also denotes lactate, formate, acetate, propionate, perchlorate, fluorinated alkyl sulfonate or fluorinated aryl sulfonate, trifluorosulfonate, trifluoromethanesulfonate (triflate), nonafluorobutanesulfonate (nonaflate), halogenated carboxylate, trifluoroacetate, trichloroacetate, dichloroacetate, monochloroacetate, tetrafluoroborate, hexafluorophosphate, and hexafluoroantimonate.

### Examples

The Examples which follow are set forth to aid in the understanding of the invention but are not intended to, and should not be construed to limit the scope of the invention in any way. The Examples do not include detailed descriptions of conventional methods, such methods are well-known to those of ordinary skill in the art.

### GENERAL EXPERIMENTAL DETAILS

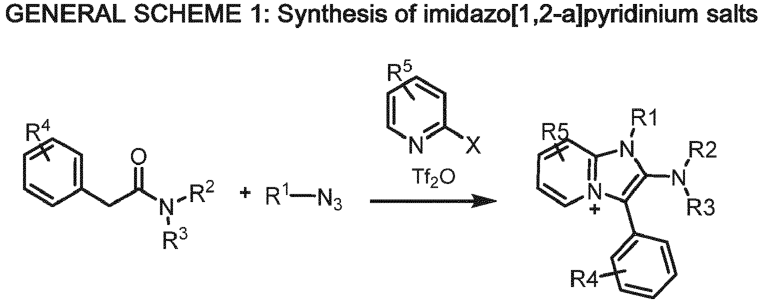

### GENERAL PROCEDURE 1 (SCHEME 1): Synthesis of imidazo[1,2-a]pyridinium salts

Based on the electrophilic activation of amides, a metal-free three-component synthesis of imidazo[1,2-a]pyridinium triflates using simple amides, azides and halopyridines as starting materials is described (SCHEME 1).

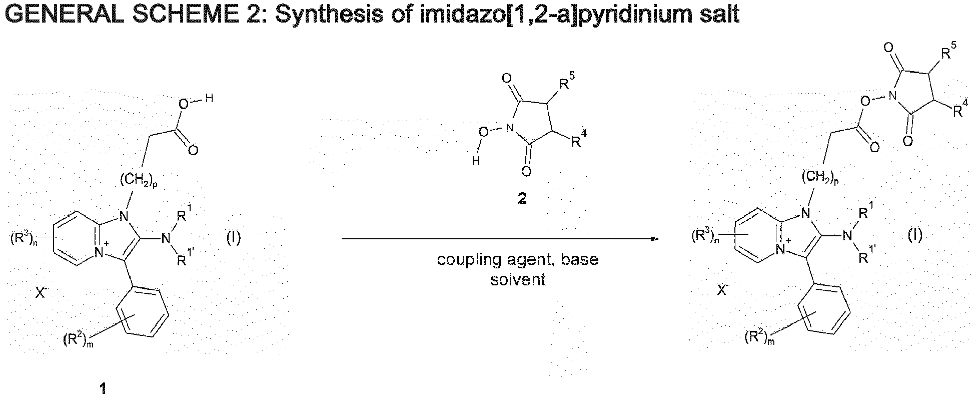

For the syntheses of compounds with the structure I, the respective imidazo[1,2-a]pyridinium salt 1 was dissolved in a solvent (e.g., dichloromethane) and to the resulting solution a coupling reagent (e.g. T3P) was added, followed by reagent **2** and a base (e.g., triethylamine). The mixture was reacted for 15 h, before work-up and purification of compound I (SCHEME 2).

The photophysical properties of this series of fluorophores were evaluated (Table 2). Spectra were measured using methanol as solvent. All substrates I-a-c present fluorescence with long wavelengths (maxima of emission λₑₘ between 510 nm and 700 nm and are excited above 400 nm. Expansion of the π-system (cf I-b vs. I-a) induces a bathochromic shift in fluorescence emission. Importantly, all these compounds display large Stoke shift (difference between λₑₘ and λ_{abs}), up to 265 nm, which helps to minimize cross-talk between the excitation source and the fluorescent emission.

**Table 2: Photophysical data of selected imidazo[1,2-a]pyridinium salts**

| **Cpds** | **λabs (nm)** | **λem (nm)** | **Stokes' Shift (nm)** | **Extinction coefficient ε _ @max absorption (1E3*M-1*cm-1)** | **Quantum yield Φ_{F}** | **Brightness @max absorption (1E3*M-1*cm-1)** |
|---|---|---|---|---|---|---|
| **I-a** | 420 | 610 | 190 | 11900 | 0.09 | 6400 |
| **I-b** | 435 | 700 | 265 | 12300 | 0.11 | 1300 |
| **I-c** | 405 | 510 | 105 | 9400 | 0.08 | 800 |

## Claims

1. A compound of formula (I), 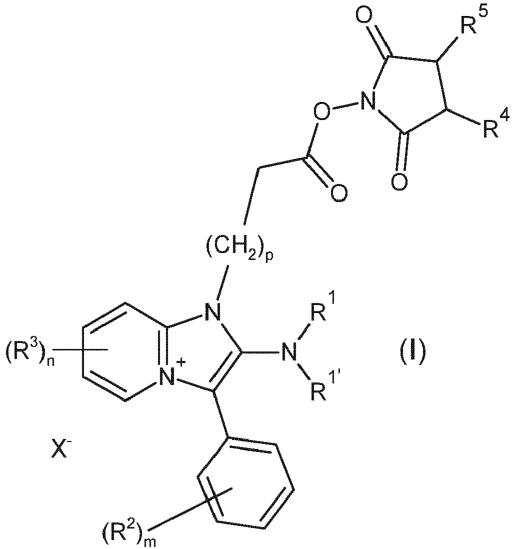 wherein
**X-** denotes an anion,
**R¹** denotes C₁₋₁₀alkyl, C₂₋₁₀alkenyl, or phenyl, optionally substituted by one or more, identical or different R^{a} and/or R^{b}, and
**R^{1'}** denotes H, C₁₋₆alkyl, or C₂₋₆alkenyl, optionally substituted by one or more, identical or different R^{a} and/or R^{b}, or
**R¹** together with **R^{1'}** form a 3 to 12 membered heterocyclic ring, and
**R²** denotes H, halogen, -SCR^{a}, -NO₂, -N3, -NR^{a}R^{a}, -P(O)(OR^{a})(OR^{a}), -S(O)₂OR^{a}, -OR^{a}, -C(O₂)R^{a}, or C₁₋₆alkyl, C₆₋₁₀aryl, optionally substituted by one or more, identical or different R^{a} and/or R^{b}, , and
**R³** denotes H, halogen, -SCR^{a}, -NO₂, -N3, -NR^{a}R^{a}, -P(O)(OR^{a})(OR^{a}), -S(O)₂OR^{a}, -OR^{a}, -C(O₂)R^{a}, or C₁₋₁₀alkyl, C₂₋₁₀alkenyl, phenyl, C₆₋₁₄aryl, 5-14 membered heteroaryl, or 5-14 membered heterocyclyl, optionally substituted by one or more, identical or different R^{a} and/or R^{b}, or
two **R³** may be bond to each other to form an aromatic ring, and
**R⁴** and **R⁵** independently from each other denote H, or halogen, or
**R⁴** and **R⁵** may be bond to each other to form an optionally substituted 5- to 6-membered cyclic group, and
**m** and **n** independently from one another denote 0, 1, 2, 3, or 4, and
**p** denotes 2, 3, 4, or 5,
each **R^{a}** independently of one another denotes H, or a group, optionally substituted by one or more, identical or different R^{b} and/or R^{c}, selected from among C₁₋₁₀alkyl, phenyl, and 3-14 membered heterocyclyl; and
each **R^{b}** is selected in each case independently of one another from among halogen, -CF3, -NO₂, =N₂, -N3, -NR^{c}R^{c}, =O, -OR^{c}, -C(O)R^{c}, -C(O)OR^{c}, -C(O)NR^{c}R^{c}, -P(O)(OR^{C})(OR^{c}), -SCR^{c}, -S(O)₂OR^{c}, and -S(O)₂R^{c}, and
each **R^{c}** independently of one another denotes hydrogen or a group, optionally substituted by one or more, identical or different R^{d} and/or R^{e}, selected from among C₁₋₁₀alkyl, C₂₋₁₀alkenyl, phenyl, and 3-18 membered heterocyclyl;
each **R^{d}** denotes a suitable substituent and is selected in each case independently of one another from among halogen, CF₃, -NO₂, =N₂, -N₃, -NR^{c}R^{c}, =O, -OR^{c}, - C(O)R^{c}, -C(O)OR^{c}, -C(O)NR^{c}R^{c}, -P(O)(OR^{C})(OR^{c}), -SCR^{c}, -S(O)₂OR^{c},
and -S(O)₂R^{c}; and
each **R^{e}** independently of one another denotes hydrogen or a group selected from among C₁₋₁₀alkyl, C₂₋₁₀alkenyl, phenyl, and 3-18 membered heterocyclyl.

2. The compound according to claim 1, wherein R¹ together with R^{1'} form a 5 membered heterocyclic ring

3. The compound according to claim 1 or 2, wherein R³ is a 5-14 membered heterocyclyl.

4. The compound according to claim 3, wherein R³ is tetrahydro-pyrido-quinoline moiety.

5. The compound according to any one of claims 1 to 4, wherein m denotes 0.

6. The compound according to any one of claims 1 to 5, wherein p denotes 3.

7. The compound according to any one of claims 1 to 6, wherein X denotes a chloride, bromide, iodide, fluoride, acetate, formiate, perchlorate, fluorinated alkyl sulfonate or fluorinated aryl sulfonate, trifluorosulfonate, trifluoromethanesulfonate (triflate), nonafluorobutanesulfonate (nonaflate), halogenated carboxylate, trifluoroacetate, trichloroacetate, dichloroacetate, monochloroacetate, tetrafluoroborate, hexafluorophosphate, or hexafluoroantimonate.

8. The compound according to claim 7, wherein X denotes trifluoromethanesulfonate (triflate).

9. The compound according to claim 1 chosen from the following Table 3:
| | | |
|---|---|---|
| 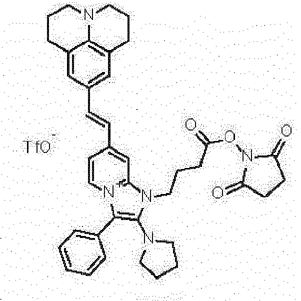 | 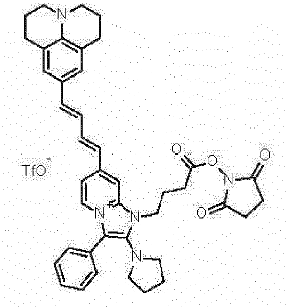 | 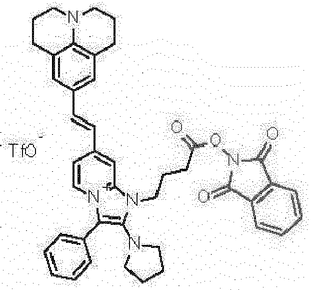 |
| I-a | I-b | I-c |

10. The compound according to any of claims 1 to 9, wherein the compounds exhibit photophysical properties, preferably fluorescence activity.

11. Use of a compound of formula (I) of any one of claims 1 to 9 as a fluorescent probe.

12. The use according to claim 11, wherein the fluorescent probe is used in the field of biology, biochemistry, chemical biology, or medical diagnostics.

13. A process for manufacturing a compound of formula (I), wherein compounds 1 and 2 are reacted in the presence of a coupling agent, a base and a solvent to obtain a compound of formula (I), 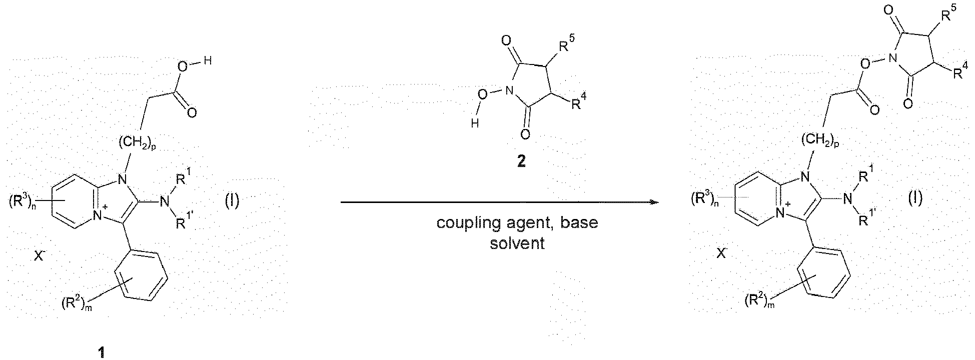 wherein
R¹, R^{1'}, R², R³, R⁴, R⁵, X-, m, n and p are as defined above.
